(19) Europäisches Patentamt
European Patent Office
Office européen des brevets

(11) **EP 2 994 177 B1**

(12) **EUROPEAN PATENT SPECIFICATION**

(45) Date of publication and mention
of the grant of the patent:
**20.09.2023 Bulletin 2023/38**

(21) Application number: **14794285.8**

(22) Date of filing: **09.05.2014**

(51) International Patent Classification (IPC):
**A61B 17/34** *(2006.01)*     **A61B 17/00** *(2006.01)*
**A61B 17/30** *(2006.01)*

(52) Cooperative Patent Classification (CPC):
**A61B 17/3417; A61B 17/3478;** A61B 2017/003;
A61B 2017/00455; A61B 2017/00845;
A61B 2017/306

(86) International application number:
**PCT/US2014/037482**

(87) International publication number:
**WO 2014/183025 (13.11.2014 Gazette 2014/46)**

(54) **TRANSJUGULAR INTRAHEPATIC PORTOSYSTEMIC SHUNT DEVICE**

TRANSJUGULÄRE INTRAHEPATISCHE PORTOSYSTEMISCHE SHUNT-VORRICHTUNG

DISPOSITIF DE SHUNT PORTOSYSTÉMIQUE INTRAHÉPATIQUE PAR VOIE TRANSJUGULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **10.05.2013 US 201313891585**

(43) Date of publication of application:
**16.03.2016 Bulletin 2016/11**

(73) Proprietor: **Hatch Medical, L.L.C.
Atlanta, GA 30324 (US)**

(72) Inventors:
• **KAHN, Sidney Lowell
Somers Connecticut 06071 (US)**
• **SEYBOLD, Brent
Santa Clara California 95051 (US)**

(74) Representative: **Gill Jennings & Every LLP
The Broadgate Tower
20 Primrose Street
London EC2A 2ES (GB)**

(56) References cited:
WO-A2-2009/091514     JP-A- 2005 177 237
US-A1- 2003 216 771     US-A1- 2005 020 963
US-A1- 2005 149 097     US-A1- 2012 283 640
US-B1- 8 287 481

**Description**

**BACKGROUND OF THE INVENTION**

Field of the Invention

The present invention relates to an apparatus for quickly locating a liver portal vein to establish a shunt through a human liver between the portal vein and the hepatic vein, and more particularly to a Transjugular intrahepatic Portosystemic Shunt ("TIPS") device.

Description of Related Art

The human liver has many functions essential to life including breaking down fats, glucose metabolism/storage, urea production, amino acid synthesis, toxin filtration, storage of vitamins and minerals, and cholesterol metabolism. On average, the healthy liver receives around 70-75% of its blood supply from the portal vein and 25-30% from the hepatic artery. Blood leaves the liver through the hepatic veins which communicate with the inferior vena cava just below the heart.

Liver cirrhosis is a term that refers to irreparable damage to the liver where the hepatic parenchyma is progressively replaced with scar tissue. With time, this can progress to complete liver failure. There are numerous causes of liver cirrhosis, but common causes include alcohol abuse, hepatitis, non-alcoholic steatohepatitis (NASH), toxin/drug exposure, and various inherited and autoimmune conditions. As the liver becomes cirrhotic, the normal blood flow patterns become altered. Specifically, the pressure within the portal veins increases causing the blood that would normally flow to the liver to "backup." Over time the portal blood that normally flows towards the liver (hepatopedal) can reverse and flow away from the liver (hepatofugal). As the portal vein pressures rise, the blood finds alternative routes to return to the heart. This typically occurs in the form of variceal vessels that are particularly prone to hemorrhage with potentially fatal consequences.

In addition, the increased portal vein pressures observed with cirrhosis raises capillary hydrostatic pressures within the splanchnic vascular bed of the viscera. Coupled with impaired renal function and sodium retention, as the hydrostatic pressure rises, a transudative fluid collection, known as ascites, can form in the patient's abdomen. Although typically this is initially managed with medical therapy, the amount of fluid can become quite large and refractory to medical therapy.

Originally described by Rosch et al. in 1969, the TIPS procedure creates a shunt between the portal veins and the hepatic veins allowing blood to bypass the cirrhotic hepatic parenchyma that is responsible for the elevated portal vein pressures. In so doing, the TIPS procedure decreases portal pressure and is indicated in the management of variceal bleeding refractory to medical therapy, refractory ascites, and refractory hepatic hydrothorax (fluid surrounding the lungs secondary to liver failure).

There are several commercially available kits for the TIPS procedure. The TIPS procedure is performed under general anesthesia by accessing the patient's right internal jugular vein and advancing a catheter through the superior vena cava, heart, and inferior vena cava to the right hepatic vein. Once catheter access to the right hepatic vein is made, a larger sheath is then placed into the right hepatic vein. Through this sheath a large needle (typically 16 Gauge) is pushed through the wall of the hepatic vein into the parenchyma anteroinferomedially in the expected direction of the right portal vein. After the needle has been advanced, a syringe is attached to the back of the needle and the needle is slowly withdrawn. When blood is aspirated, a small injection of contrast is made into the needle to confirm access into the right portal vein. Wire access through the needle into the right portal vein and main portal vein is then made. Over this wire, a tract is balloon dilated and a covered stent is placed thus forming the shunt.

Despite the use of preprocedural imaging as well as intraprocedural wedge portography, access to the portal vein can often prove difficult requiring numerous passes of the needle before access is obtained. The passage of the needle through the liver is associated with complications including intraperitoneal bleeding (frequency 1-13%), hemobilia (frequency 1-4%), and fistula formation (frequency < 1%). Equally important, the difficulty of portal vein access extends procedure time with the increased use of hospital resources/expenses and the added risk to the patient of prolonged anesthesia and fluoroscopic radiation exposure. Therefore, there is a need for a TIPS device and method that can obtain access to the portal vein more safely and rapidly.

US 2012/283640 A1 discloses an access device for placing a medical article within a body space.

**SUMMARY OF THE INVENTION**

The invention is defined in the independent claim and other embodiments are listed in the dependent claims.

An embodiment includes an insertion apparatus comprising an outer guide with either a straight (discussed in the next paragraph) or steerable inner needle. The assembled apparatus with the steerable needle allows the needle to be directed in the same direction as the outer guide. The steerable needle can be directed in any direction along a 360 degree arc. The employment of a steerable inner needle enhances the ease of portal vein catheterization by allowing the operator to steer the needle in the expected direction of the portal vein. This confers advantages including decreased procedure time and enhanced safety. The reduced procedure time confers advantages of cost-savings, decreased anesthesia risk to the patient, and decreased radiation exposure to both the patient and the operator. A further embodiment of this apparatus includes the substitution of the curved inner needle (which allows steering) for a straight inner needle (non-steerable). This would be used in the event that the operator believes the portal vein lies directly in front of the curved outer guide. In such a situation a straight needle would be optimal for portal vein catheterization as it provides a more direct route.

In some embodiments, this apparatus comprises an elongated hollow outer guide; an outer handle having an inner lumen and a first luer lock, the outer handle attached at a proximal end of the outer guide, the outer guide in flow communication with the inner lumen and the luer lock; an elongated hollow inner needle; and a hub having a second luer lock, the hub attached at a proximal end of the inner needle, the inner needle in flow communication with the second luer lock. The inner needle is slidingly received through the first luer lock and into the outer guide through the inner lumen. The inner needle is configured to rotate within the outer guide by manipulation of the hub; and a distal tip of the inner needle is deployed out of and beyond a distal tip of the outer guide, the distal tip of the inner needle configured to be advanced into the parenchyma of the liver to locate the portal vein. To assist in located the portal vein, the inner needle is capable of aspirating fluid therethrough.

The present disclosure also comprising an example method not forming part of the invention of locating a liver portal vein necessary for performing a transjugular intrahepatic portosystemic shunt procedure utilizing the outer guide/inner needle device, comprising catheterizing, through the jugular vein, a liver hepatic vein of a patient receiving the transjugular intrahepatic portosystemic shunt. Inserting the device into the jugular vein and through to the liver hepatic vein. Rotating the outer handle of the device until the outer guide is in the expected direction of the liver portal vein. Stabilizing the outer handle and rotating the inner needle by way of the hub in the expected direction of the liver portal vein. Applying pressure to the hub and advancing the distal tip of the inner needle into the parenchyma of the liver, through the wall of the hepatic vein in a direction towards the portal vein. Creating an aspirating force inside the inner needle to locate the liver portal vein and establishing a shunt to the portal vein.

Consequently, the devices described herein will result in considerable cost savings and improved safety by shortening the length of time of the procedure. The decrease in procedural time is significant as it lowers the rate of complications related to the administration of general anesthesia. Moreover, the decrease in procedural time offers cost savings benefits to the healthcare system.

In another embodiment, the device employs 6 smaller needles that extend from the distal end of a TIPS needle.

In yet another embodiment, the device employs 8 smaller needles that extend from the distal end of a TIPS needle.

In yet still another embodiment, the device employs only one sheath in which the TIPS needle passes through.

In yet still another embodiment, the device employs an outer guide receiving a steerable curved inner needle and an apparatus for the manipulation of the inner needle to locate the portal vein during a TIPS procedure.

It is an object of the present disclosure to provide a multi-needled TIPS device that can obtain access to the portal vein from the hepatic vein more safely and rapidly.

It is another object of the present disclosure to provide an example method not forming part of the invention for using the multi-needled TIPS device to reduce the time necessary to establish a shunt.

In accordance with these and other objects which will become apparent hereinafter, the instant invention will now be described with particular reference to the accompanying drawings.

## BRIEF DESCRIPTION OF THE DRAWINGS

Figure 1 is a drawing to illustrate a human liver, the hepatic vein, and the portal vein.

Figure 2 is a side cross-sectional view in elevation in a retracted state.

Figure 3 is a side cross-sectional view in elevation in an extended state.

Figure 4 is a side elevational view partially cut away of an outer sheath.

Figure 5 is a side elevational view partially cut away of an inner sheath.

Figure 6 is a side elevational view of a TIPS needle in a retracted state.

Figure 7 is a side view of a TIPS needle with four engaging needles in an extended state and the tip of the TIPS needle in phantom.

Figure 8 is a perspective view partially cutaway of the distal end of a TIPS needle.

Figure 9 is a side elevational view of the distal end of a TIPS needle with a needle stylets in partial phantom.

Figure 10 is a side elevational view of engaging needles and a common needle hub.

Figure 11 is a side elevational view of stylets and a common stylet hub.

Figure 12 is a side elevational view in cross section of an aspirating syringe.

Figure 13 is a perspective elevational view of an embodiment of the present invention, in a retracted state.

Figure 14 is a perspective elevational view of an embodiment of the present invention, in an extended state also showing the hub cab removed from the hub.

Figure 15 is a close-up perspective elevational view of another embodiment of the present invention.

Figure 16 is a perspective elevational view of the outer guide used in one embodiment of the present invention.

Figure 17 is a perspective elevational view of the inner needle used in one embodiment of present invention.

Figure 18 is a perspective elevational view of the inner needle hub used in one embodiment of the present invention.

Figure 19 is a perspective elevational view of the hub cap used in one embodiment of the present invention.

## DETAILED DESCRIPTION OF THE INVENTION

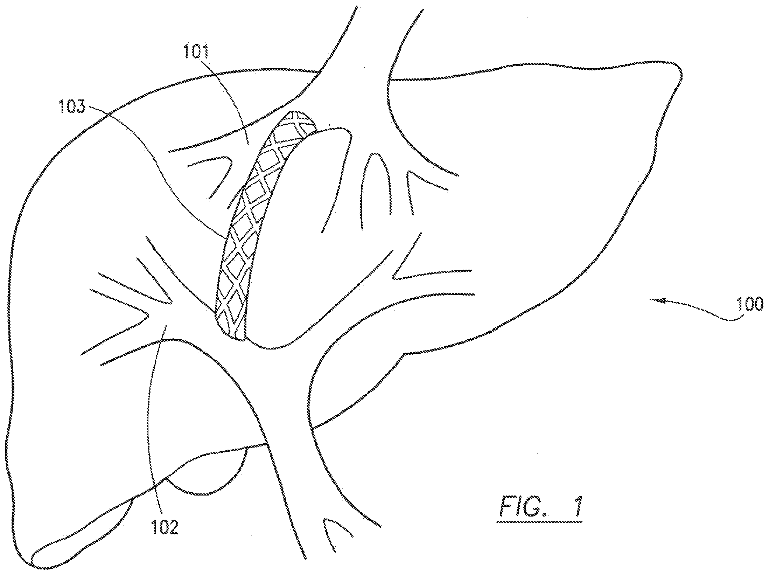

A multi-needle TIPS device and an example method not forming part of the invention for using the multi-needle TIPS device, are described with initial reference to Figure 1. The device and method described herein is desirable for locating quickly the proper portal vein for establishing a shunt 103 through a human liver 100, connecting the right hepatic vein 101 to the right portal vein 102.

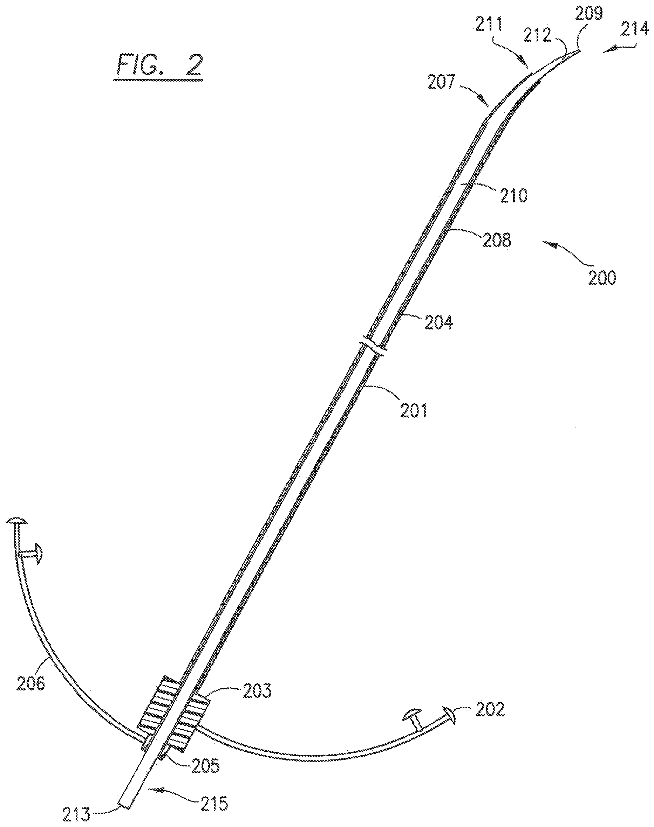

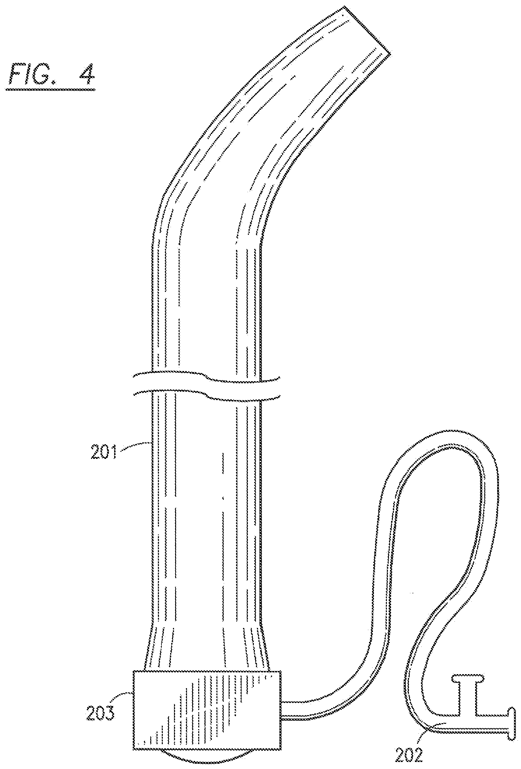

Referring now to Figures 2, 3, 4, 5, 6, and 7, the device 200 and its primary components are depicted in isolation and in a retracted and extended state. Figure 2 shows the device 200 in the retracted state, which is a non-engaging, default state. An outer sheath 201 is the primary exterior component of the device 200. The outer sheath comes with an inner dilator to allow introduction over a wire into the right hepatic vein. The outer sheath 201 is a ten (10) French (for reference, 1 French = 3 mm) sheath which is made of a semi-rigid material. In Figure 4, the outer sheath 201 has a shaft that measures between 35 and 40 cm in length and an outer hub 203 with an outer side port 202 at its proximal end that is designed to allow flushing of the outer sheath 201.

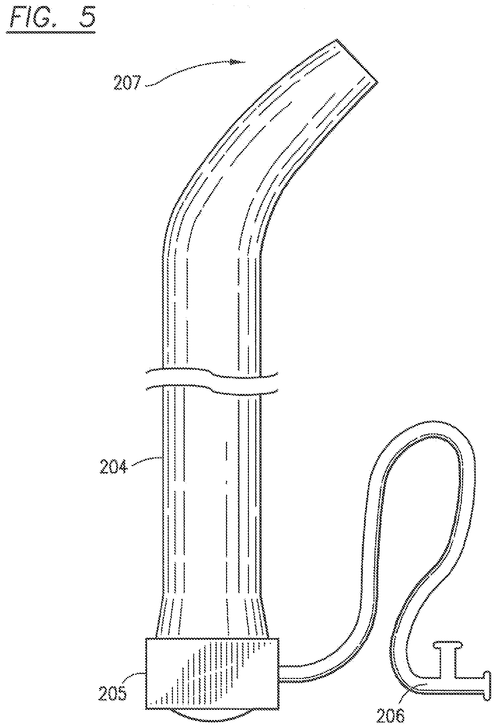

In Figure 5, the lumen of the outer sheath 201 accommodates an inner sheath 204. The inner sheath 204 is an eight (8) French sheath which is made of a semi-rigid material. The shaft of the inner sheath measures 45 cm in length has a tapered end 207. The inner sheath 204 also has an inner hub 205 and inner side port 206 at its proximal end to allow for flushing of the inner sheath 204. The shaft of the inner sheath 204 also has a braided metallic wall to allow passage of an 8 French needle without damage to the inner sheath 204.

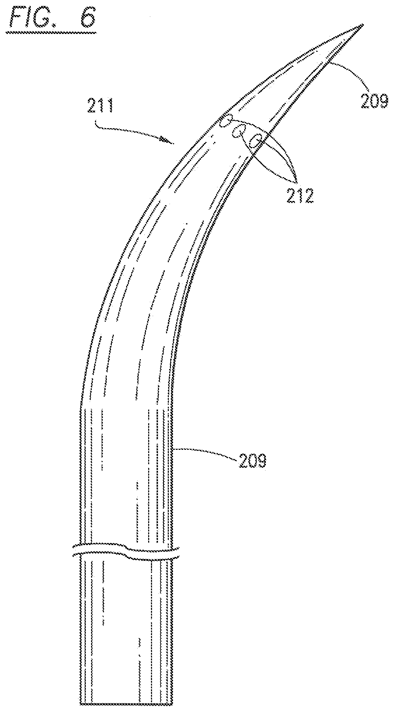

The lumen of the inner sheath 204 accommodates a TIPS needle 208, which in total measures 50 cm in length, including a curved portion 211 of 7 cm at the TIPS needle's distal end 214. The TIPS needle is an eight (8) French needle made of a semi-rigid material. The curved portion 211 has a 30 degree curve. At distal end of the TIPS needle 208 is a TIPS needle tip 209 (See Figure 6). Near the distal end of the TIPS needle 208, before the TIPS needle tip 209, there are four needle holes 212 designed to allow smaller needles to pass through. The TIPS needle also has a TIPS needle hub 213 at the proximal end 215 of the TIPS needle.

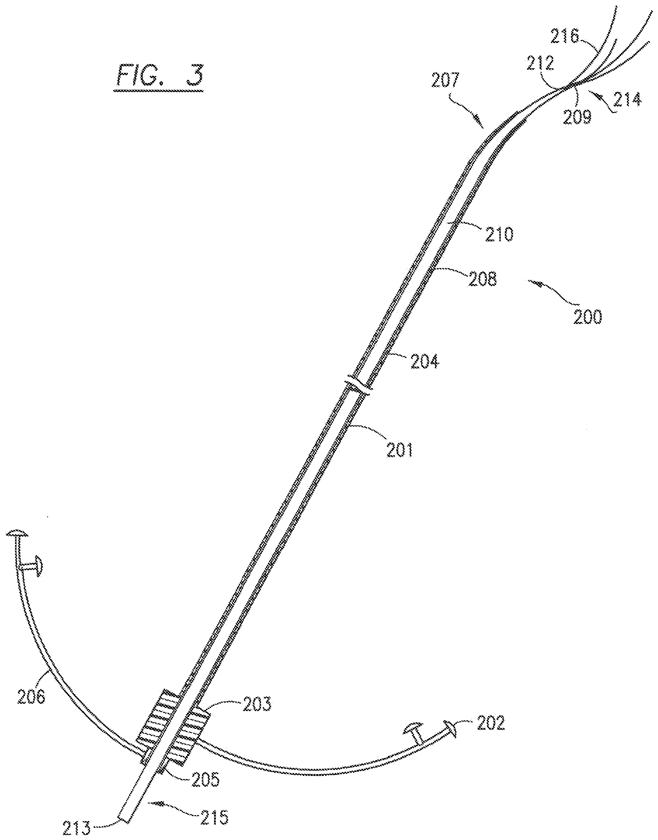

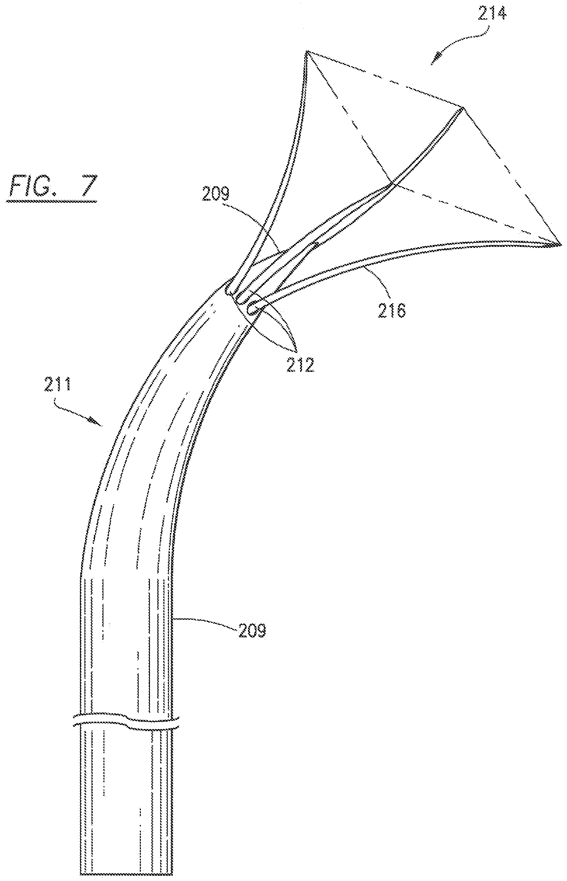

Referring back to Figure 3 shows the device 200 depicted in the extended state. The extended state is the engaging state and is defined by the extension of four (4) engaging needles 216 from the distal end 214 of the TIPS needle 204. The engaging needles 216 are each twenty-three (23) gauge needles made from a semi-rigid material and each has a preformed gentle curve in its tip. The engaging needles 216 pass through the TIPS needle lumen 210, with each engaging needle 216 extending from one of the four needle holes 212 of the TIPS needle 208. Inside the TIPS needle lumen 210, the engaging needles 216 are separated by thin horizontal and vertical septations 218 in the TIPS needle lumen 208. As seen in Figure 7, while extending, the engaging needles 216 progressively spread apart in a square pattern. When fully extended, the engaging needles 216 lie in a square pattern with 2-3 cm between the needles.

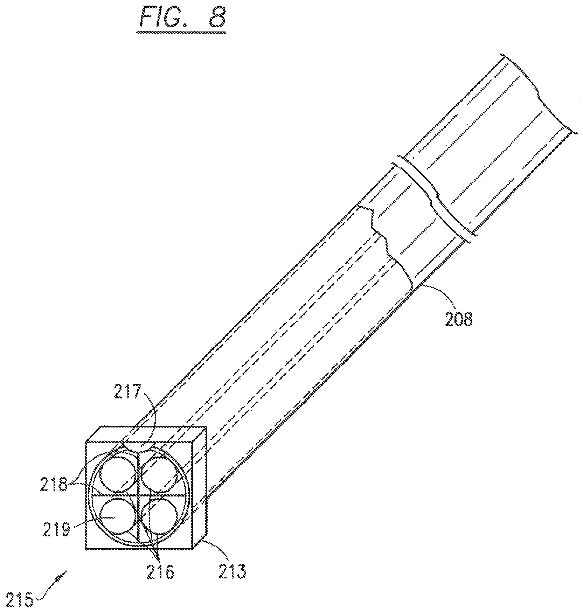

Referring now to Figures 8, 9, 10, and 11, the primary components that comprise the interior of the device are shown. Figure 8 shows the proximal end 215 of the TIPS needle 208. At the proximal end 215 of the TIPS needle 208 is the TIPS needle hub 213. The TIPS needle hub 213 serves as the base of the TIPS needle 208 and houses the hub adapter 217. As a result of the horizontal and vertical sepations 218 in the TIPS needle lumen 208, the TIPS needle lumen 208 contains four distinct chambers, each of which can accommodate one engaging needle 216. The engaging needles are each attached to a common needle hub 223, which allows the engaging needles to be advanced and retracted together. To assure that proper pattern of the engaging needles 216 when they are extended from the TIPS needle 208, each engaging needle 216 has a specific target chamber within the TIPS needle 208. If the engaging needles 216 are inserted into the proper chamber, then once the engaging needles 216 are completely advanced into the TIPS needle 208, the common needle hub 223 will engage the hub adapter 217.

Stylets 221, are used to fill the engaging needle lumen 219 and provide additional rigidity to the engaging needles 216 when they are advanced into the engaging needle lumen 219. The stylets 221 are of twenty-three 23 gauge size, made from a semi-rigid material, and are all attached to a common stylet hub 222. In addition, the stylets 221 have the same preformed curve as the engaging needles 216. The stylets 221 are utilized by insertion first through a sliding cover 220, then through the common needle hub 223, and finally through the engaging needle lumen 219.

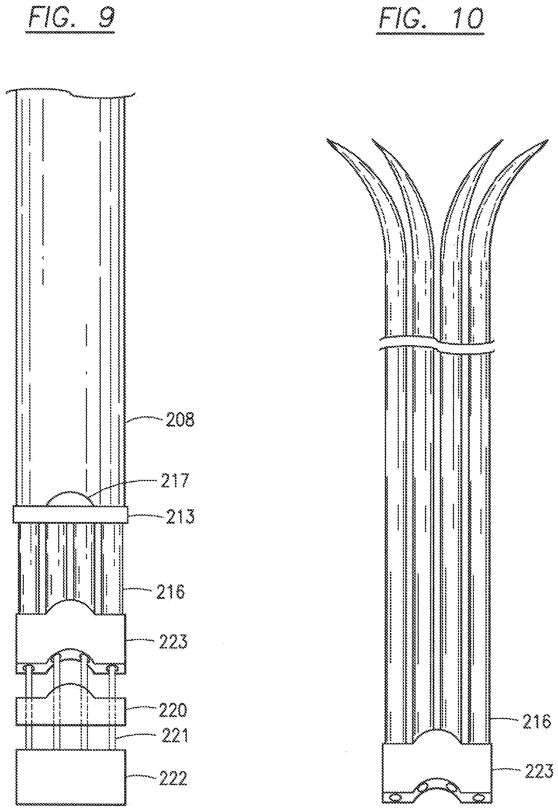

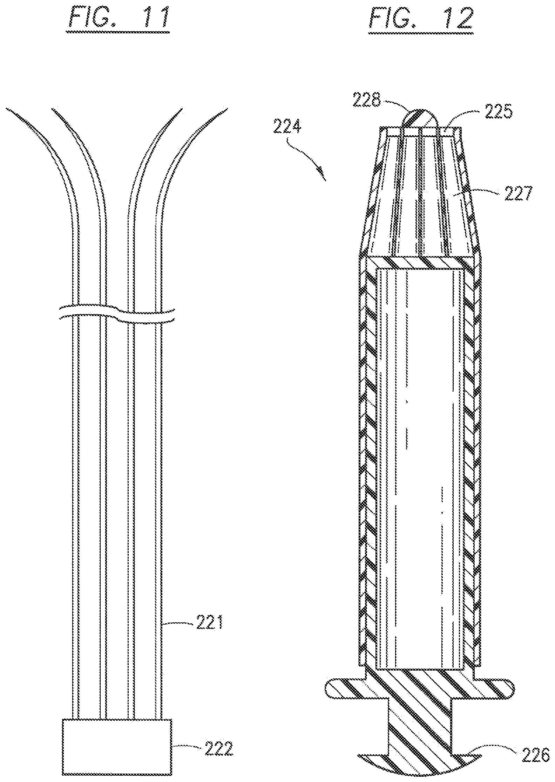

Referring now to Figure 12, a specially designed syringe 224 is shown. The syringe 224 is a sixty (60) cc syringe that is designed to engage the common needle hub 223 (Figure 9) and provide aspiration through the engaging needle lumen 219. The syringe has an engaging tip 225 with four independent syringe chambers 227, where each chamber 227 corresponds to one of the four engaging needle lumen 219 (Figure 8). Along with an opening for each syringe chamber 227, the engaging tip 225 also houses a syringe adapter 228 that fits the common needle hub 223. When syringe 224 is engaged to the common needle hub 223 and the plunger 226 of the syringe 224 is pulled, aspiration is provided through each of the engaging needle lumen 219.

The operation of the device begins with the access of the right hepatic vein 101 (Figure 1) from the right jugular vein. Under ultrasound guidance, a twenty-one (21) Gauge needle will be used to access the right jugular vein. After obtaining access, a 0.018" wire is advanced through the lumen of the twenty-one (21) Gauge needle and its position confirmed in the superior vena cava with fluoroscopy. A transitional dilator is used to upsize the wire. Catheterization of the right hepatic vein 101 is then performed. Wedge hepatic pressures and portography are performed as needed. The outer sheath 201 and inner dilator are advanced over the wire into the right hepatic vein 101. The inner dilator is then removed and the outer side port 202 is connected to a heparinized saline flush.

Next, the TIPS needle 208 is partially inserted into the inner hub 205 and then the inner sheath 204, so as not to extend the TIPS needle tip 209 beyond the distal end of the inner sheath 204. The assembled apparatus of the TIPS needle 208 and the inner sheath 204 is then inserted into the outer hub 203 and then the outer sheath 201 and extended to the wall of the right hepatic vein 101. Once placed, the inner side port 206 can be connected to a heparinized saline flush as needed.

The engaging needles 216 with their stylets 221 seated are then advanced into the TIPS needle hub 213, but the tips of the engaging needles 216 are not extended through the holes near the end of the TIPS needle 208. At this point, the assembled device will be as shown in Figure 2. Next, the TIPS needle 208 is slowly advanced a short distance into the hepatic parenchyma anteromedially in the expected direction of the right portal vein 102. The distance that the TIPS needle is advanced into the hepatic parenchyma will likely be around 1 cm, unlike the usual distance of approximately 5 cm that is employed with a typical TIPS apparatus.

Once the TIPS needle 208 is seated in proper position, the engaging needles 216 with their inner stylets 221 are slowly advanced into the hepatic parenchyma. At this point, the assembled device will be as shown in Figure 3. The engaging needles 216 are advanced approximately 3-6 cm. As the engaging needles 216 are advanced through the needle holes 212 near the end of the TIPS needle 208 into the hepatic parenchyma they will gradually spread apart in a square shape, as depicted in Figure 7. When fully extended, the tips of the engaging needles will lie in a square pattern with 2-3 cm between the needles. Since the needles progressively spread with further advancement, extending the engaging needles 216 a shorter distance will result in the needle tips lying closer to one another in a smaller square pattern.

The stylets 221 are removed from the engaging needle lumen 219 by pulling on the common stylet hub 222. As the stylets 221 are withdrawn from the engaging needles 216, their tips are covered by the needle cover 220. This needle cover 220 is designed to prevent a needle injury to the operator and to keep the stylet tips together when disengaged from the engaging needles 216. The stylets 221 with their tips covered by the needle cover 220 can then be placed to the side.

With the stylets removed, the syringe 224 is then attached to the common needle hub 223 with each syringe chamber 227 corresponding to one engaging needle lumen 219. The operator then pulls on the plunger 226 to cause an aspirating force through the engaging needle lumen 219 and the tips of the engaging needles 216. The engaging needles 219 are then slowly retracted through the hepatic parenchyma while being aspirated with the syringe 224 by pulling on the syringe 224 together with the common needle hub 223. As the operator retracts the engaging needles 216, the individual syringe chambers 227 are carefully inspected for the presence of portal blood. If blood is not aspirated, the engaging needles 216 can be withdrawn into the TIPS needle 208 and the stylets 221 are replaced into the engaging needle 216. The engaging needles 216 are then advanced in a different direction and the process is repeated until aspiration of blood is successful.

When blood is aspirated into one of these syringe chambers 227, retraction of the engaging needles 216 is immediately stopped. The syringe chamber 227 and corresponding engaging needle lumen 219 containing blood are noted. The syringe 224 is then removed from the common needle hub 223. A small injection of contrast is then made into the engaging needle lumen 219 from which blood was aspirated. This is performed under fluoroscopy to confirm access into the right portal vein 102. Next, under fluoroscopy, a 0.018" wire is advanced into the appropriate engaging needle lumen 219 that had aspirated blood. This wire is advanced well into the right portal vein 102 and then into the main portal vein, splenic vein, or superior mesenteric vein as is necessary to obtain adequate purchase for further intervention.

$$1'' \text{ (inch)} = 25{,}4 \text{ mm } \P$$

Once adequate purchase is obtained, the TIPS needle 208 and the attached engaging needles 216 are pulled back as a unit together over the wire. Serial dilatation is then performed over the 0.018" wire and the 0.018" wire is upsized for a larger 0.035" wire. Over the 0.035" wire balloon angioplasty and placement of a covered stent can be performed as is done with a typical TIPS procedure. The procedure is completed with a repeat portogram and measurement of portal pressures as well as potential embolization of varices as required.

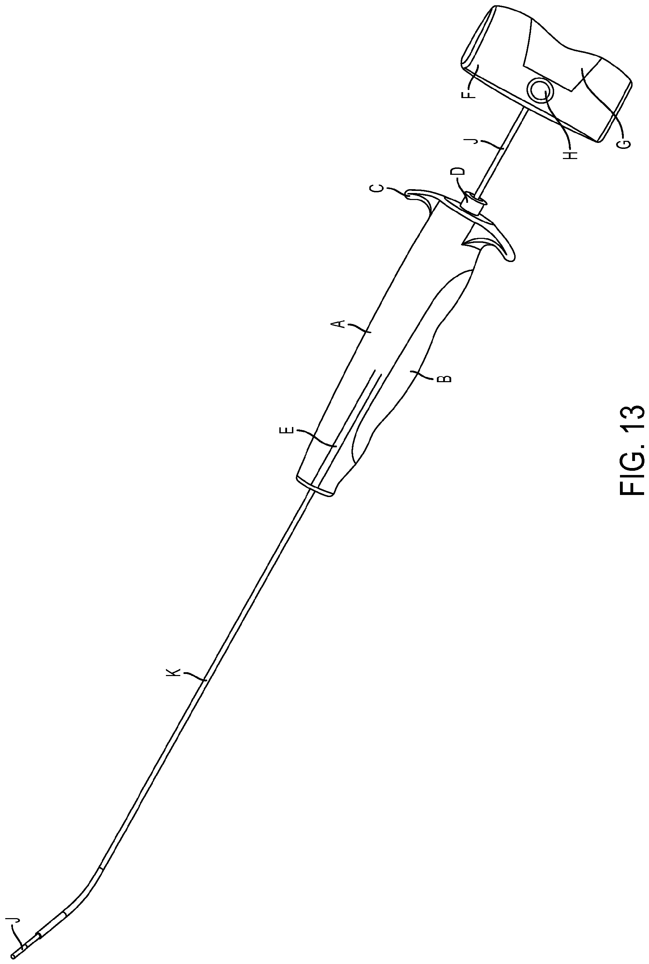

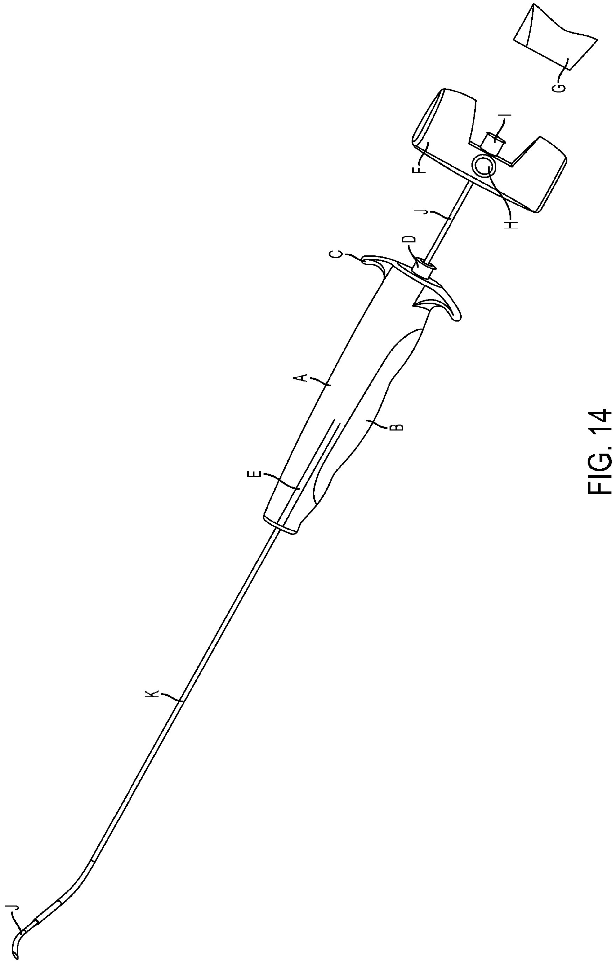

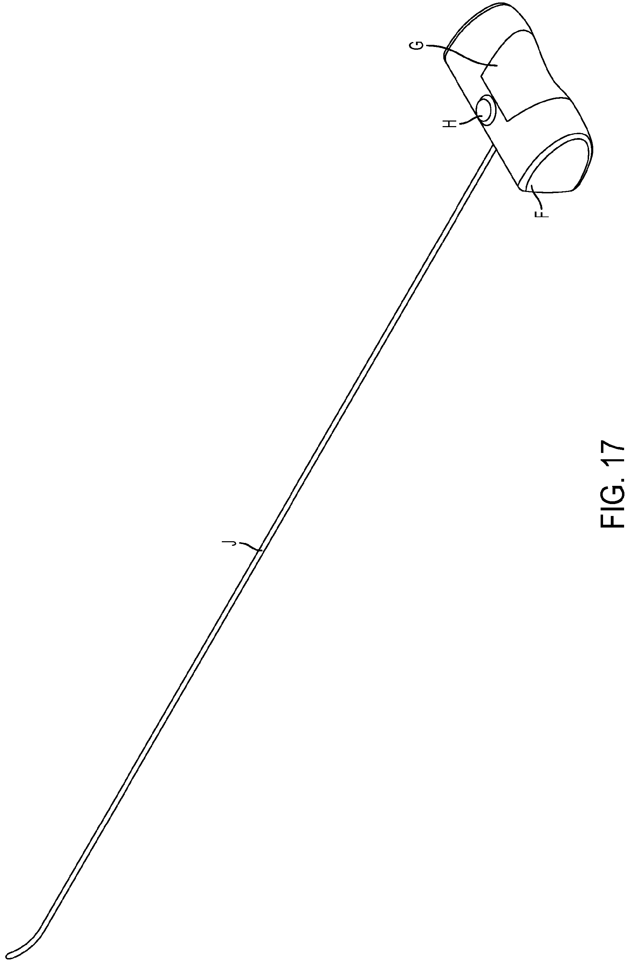

With reference to FIGS. 13-19, shown is an embodiment of the present invention providing another suitable and improved means of locating and accessing the portal vein as part of a TIPS procedure.

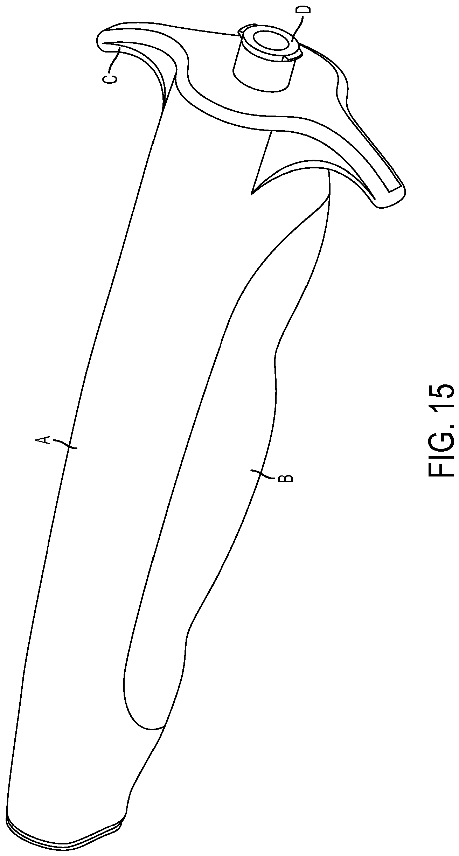

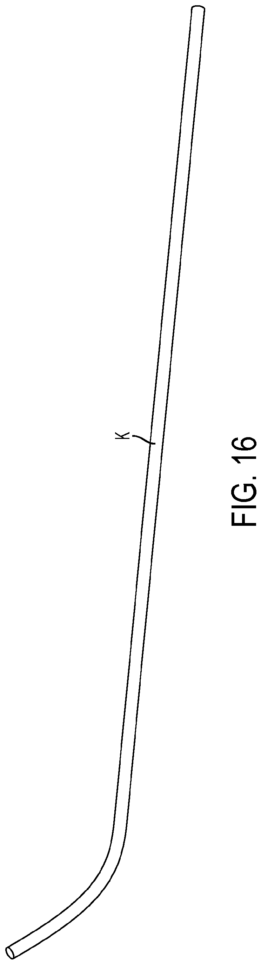

The assembled apparatus shown in Figure 13 depicts one embodiment of the present invention in an assembled state. An outer handle A (shown alone in Figure 15) is connected to the outer guide K (shown alone in Figure 16). The outer handle A has finger grips B along its inferior edge. These grips B allow the operator to torque the outer guide K as desired with one hand (typically the left hand). The outer handle A also has finger winglets C. The winglets are used by the opposite hand (typically the right hand) to assist in the deployment of the inner needle J by providing leverage from which the base of the hand can be pushed against the inner needle hub F. The proximal end of the outer guide K has a luer lock D, which not only allows insertion of the inner needle J into the outer guide K, but also allows attachment of a standard syringe thereto for aspiration or injection as needed. The inner lumen E is disposed within the outer handle A and extends therethrough in communication with the outer guide K and luer lock D. In some embodiments, the inner lumen E is just large enough to accommodate passage of a 4 French catheter. The lumen E of the outer handle is in continuity and flow communication with the outer guide K and the luer lock D. In some embodiments, the outer guide K, will measure between 40 and 50 cm and will have a gentle curve at its distal end producing an arc between 20 and 45 degrees. In some embodiments, the end of the outer guide K is flush with no bevel.

The inner needle J is slidingly and removably received in the outer guide, provided through luer lock D, through lumen E and into the outer guide K. At the proximal end of inner needle J is hub F. The hub F has a hub cap G which covers a luer lock I which provides a passage between the hub F and the inner lumen of the inner needle J. This luer lock will similarly allow not only passage of a 0.035 inch wire through the hub F and into the inner needle J, but also will allow attachment of a standard syringe for aspiration or injection as needed.

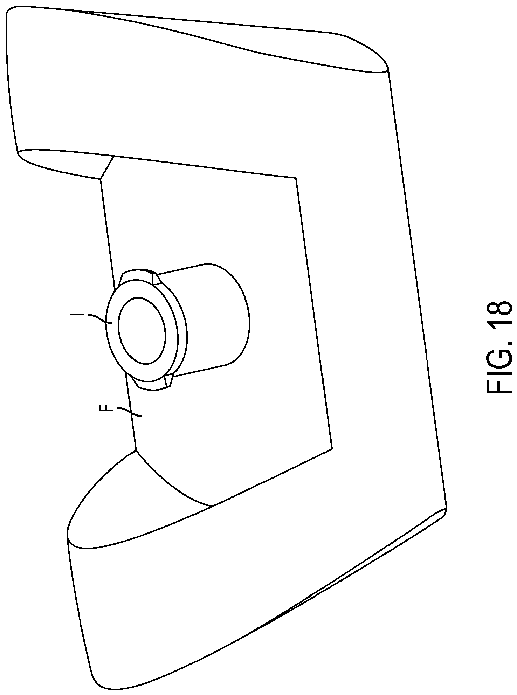

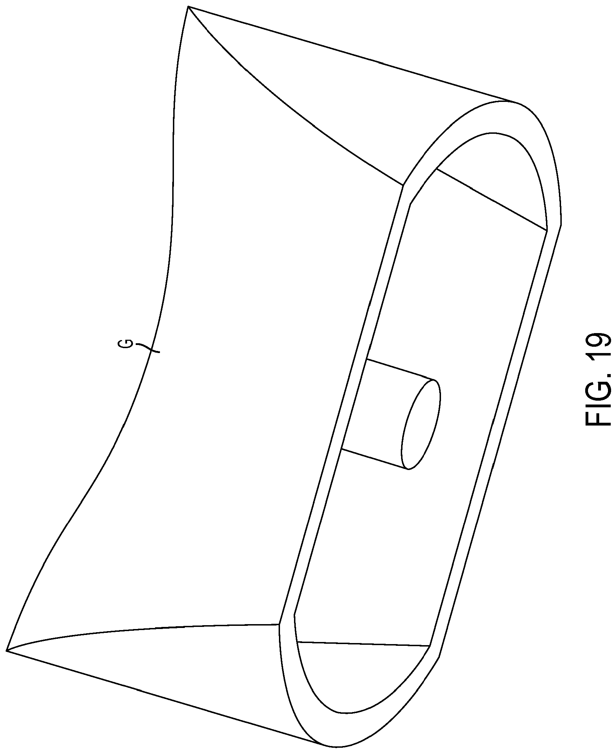

The inner needle hub F and the hub cap G are depicted in isolation in Figures 18 and 19 respectively, with luer lock I shown in Figure 18, protruding from the hub F.

The inner needle hub F allows the operator to turn and torque the inner needle J in a 360 degree radius within the outer guide K. This is performed in a 1:1 fashion as the hub F is fixed to the proximal end of the inner needle J. The inner needle hub F has a marker H, which delineates the direction of the curve of the inner needle J so that the operator understands the direction the inner needle J is pointing. This allows the user to manually determine the orientation of the distal tip of the inner needle J without having to rely on imaging such as fluoroscopy.

In some embodiments, the inner needle J will measure between 45 and 55 cm in length and will have a gentle curve at its distal end producing an arc between 20 and 45 degrees. Accordingly, the distal end of the inner needle J will substantially match the curvature and profile of the distal end of the outer guide K. A straight version of the inner needle J with the same length is an alternative embodiment of the present device. Both the straight and curved versions of the inner needle J will have a beveled tip to provide the necessary sharpness to penetrate the parenchyma of the liver. This is dissimilar from the non-beveled distal tip embodiment of the outer guide K, since the outer guide K would not need to be used to penetrate the liver.

In some embodiments, the inner needle J has a hydrophilic coating or Teflon coating to reduce the friction between the inner needle J and the outer guide K. As an alternative embodiment of the device, the inner needle may have a 4 French plastic polymer sleeve/catheter around the outside of the inner needle J. Such a sleeve/catheter will have a luer lock at the end (allowing it to lock to the inner needle hub F), and will closely resemble a standard diagnostic angiographic catheter. The sleeve/catheter will advance with the inner needle J during use of the device. The purpose of the sleeve/catheter is to reduce friction as well as facilitate catheterization of the portal vein and advancement of the wire once the portal vein is found with the inner needle J.

The operation of this embodiment of the present invention is as follows: As with any standard TIPS procedure, the goal is to create a shunt between the hepatic vein and portal vein using a covered stent as a conduit for blood flow through the liver. This is demonstrated in Figure 1 where blood flows from the portal vein, 102, to the hepatic vein, 101. Typically this involves communication between the right hepatic vein and the right portal vein, but variations on this exist depending upon the patient's anatomy and the need for additional shunting.

Using standard angiographic technique, catheterization of the right jugular vein, the inferior vena cava, and then hepatic vein (typically right) is obtained. After confirming appropriate hepatic vein catheterization, a stiff wire is placed distally within the vein. Over the stiff wire, a 10 French sheath is advanced well into the hepatic vein. At this point, wedged portal pressures and wedged portography with an occlusion balloon can be performed at the operator's discretion to delineate the location of the target portal vein and assess the degree of portal hypertension.

The apparatus of the present invention is then inserted through the sheath and is advanced to the tip of the sheath. The assembled apparatus is advanced such that the inner needle J and hub F are retracted so that the sharp tip of the inner needle J initially does not extend beyond the tip of the outer guide K. Once the distal tip of the assembled apparatus reaches the tip of the sheath, the sheath is gently withdrawn to expose the outer guide K.

Using the outer handle A the apparatus is rotated in the general expected direction of the target portal vein. The operator then stabilizes the outer handle A and therefore outer guide K, with his or her left hand. The inner needle hub F is then rotated with the operator's right hand in the expected direction of the target portal vein to fine tune the targeting of the portal vein. Using the right hand, two fingers are placed on the finger winglets C. The base of the right hand is then used to apply pressure to the base of the inner needle hub F, advancing the distal tip of the inner needle J into the liver parenchyma.

The hub cap G of the inner needle hub F is then removed, exposing the luer lock I of the needle hub F. A saline containing syringe is attached to luer lock I and a small amount is injected to clear the lumen of the inner needle J. The saline syringe is removed, and a contrast dye syringe is attached. A combination of gentle aspiration and short puffs of contrast dye are made while the inner needle J and inner needle hub F are slowly retracted back towards the outer guide K. Once the portal vein is found, a wire can be directly advanced through the lumen of the inner needle J into the portal vein or alternatively, the sleeve/catheter of the inner needle J can be advanced over the inner needle, J into the portal vein at which time the inner needle J and inner needle hub F can be removed. If the portal vein is not found on initial deployment of the inner needle J the hub cap G can be replaced, and then the inner needle J and inner needle hub F can be rotated to a new orientation and the process repeated until the portal vein is found.

Regardless of the exact technique, once a wire is successfully advanced into the portal vasculature, the remainder of the TIPS procedure is completed with standard technique. This requires advancement of a 4 French catheter over a wire and through the outer guide handle A, and outer guide K, into the portal vein. The initial wire advanced is then exchanged for a stiffer wire over which a diagnostic 4 French flush catheter can be advanced and used to perform portography and obtain portal pressures. The stiff wire is replaced and the diagnostic flush catheter removed. Now, the outer guide K and outer handle A can be safely removed. The tract through the liver parenchyma is then balloon dilated until the 10 French sheath can be successfully advanced from the hepatic vein through the liver parenchyma into the portal vein. Once the sheath is advanced into the portal vein, the TIPS stent is deployed per the manufacturer's IFU and the diameter adjusted with balloon angioplasty until the desired portosystemic gradient is obtained.

The instant invention has been shown and described herein in what is considered to be the most practical and preferred embodiment. It is recognized, however, that departures may be made there from within the scope of the appended claims and that obvious modifications will occur to a person skilled in the art.

**Claims**

1. A device used for establishing a transjugular intrahepatic portosystemic shunt quickly by locating a portal vein in a liver for receiving the shunt comprising:

   an elongated hollow curved outer guide (K);
   an outer handle (A) having an inner lumen (E) and a first luer lock (D), the outer handle (A) attached at a proximal end of the outer guide (K), the outer guide (K) in flow communication with the inner lumen (E) and the first luer lock (D);
   an elongated hollow curved inner needle (J);
   a hub (F) having a second luer lock (I), the hub (F) attached at a proximal end of the inner needle (J), the inner needle (J) in flow communication with the second luer lock (I);
   wherein the inner needle (J) is slidingly received through the first luer lock (D) and into the outer guide (K) through the inner lumen (E);
   wherein the inner needle (J) is configured to rotate within the outer guide (K) by manipulation of the hub (F);
   wherein a distal tip of the inner needle (J) is deployed out of and beyond a distal tip of the outer guide (K), the distal tip of the inner needle (J) configured to be advanced into the parenchyma of the liver to locate the portal vein; and
   wherein the outer handle (A) has finger grips (B) along its inferior edge to allow an operator to torque the outer guide, the device further comprising finger winglets (C) on the outer handle (A) to assist in deployment of the inner needle (J).

2. The device of claim 1, wherein the inner needle is capable of aspiring fluid therethrough.

3. The device of claim 1, further comprising a hub cap (G) removably received on the hub (F).

4. The device of claim 1, further comprising a marker (H) on the hub (F) providing an indication of the orientation of the inner needle (J).

5. The device of claim 1, wherein the distal tips of the outer guide (K) and the inner needle (J) have matching curved geometry.

**Patentansprüche**

1. Eine Vorrichtung, die zum schnellen Anlegen eines transjugulären intrahepatischen portosystemischen Shunts durch Lokalisieren einer Portalvene in einer Leber zum Aufnehmen des Shunts verwendet wird, die Folgendes beinhaltet:

eine längliche hohle gekrümmte äußere Führung (K);
einen äußeren Handgriff (A) mit einem Innenlumen (E) und einem ersten Luer-Lock (D), wobei der äußere Handgriff (A) an einem proximalen Ende der äußeren Führung (K) angebracht ist, wobei die äußere Führung (K) in Fließverbindung mit dem Innenlumen (E) und dem ersten Luer-Lock (D) steht;
eine längliche hohle gekrümmte innere Nadel (J);
einen Ansatz (F) mit einem zweiten Luer-Lock (I), wobei der Ansatz (F) an einem proximalen Ende der inneren Nadel (J) angebracht ist, wobei die innere Nadel (J) in Fließverbindung mit dem zweiten Luer-Lock (I) steht;
wobei die innere Nadel (J) gleitend durch den ersten Luer-Lock (D) und durch das Innenlumen (E) in die äußere Führung (K) aufgenommen wird;
wobei die innere Nadel (J) dazu ausgelegt ist, durch Manipulation des Ansatzes (F) innerhalb der äußeren Führung (K) zu rotieren;
wobei eine distale Spitze der inneren Nadel (J) aus und jenseits einer distalen Spitze der äußeren Führung (K) abgelegt wird, wobei die distale Spitze der inneren Nadel (J) dazu ausgelegt ist, in das Parenchym der Leber vorgeschoben zu werden, um die Portalvene zu lokalisieren; und
wobei der äußere Handgriff (A) Fingermulden (B) entlang seines unteren Rands aufweist, um es einem Bediener zu gestatten, die äußere Führung festzudrehen, wobei die Vorrichtung ferner Fingerflügelchen (C) auf dem äußeren Handgriff (A) beinhaltet, um beim Ablegen der inneren Nadel (J) zu helfen.

2. Vorrichtung gemäß Anspruch 1, wobei die innere Nadel in der Lage ist, Fluid durch sich zu aspirieren.

3. Vorrichtung gemäß Anspruch 1, die ferner eine Ansatzkappe (G) beinhaltet, die entfernbar auf dem Ansatz (F) aufgenommen wird.

4. Vorrichtung gemäß Anspruch 1, die ferner einen Marker (H) auf dem Ansatz (F) beinhaltet, der einen Hinweis auf die Ausrichtung der inneren Nadel (J) bereitstellt.

5. Vorrichtung gemäß Anspruch 1, wobei die distalen Spitzen der äußeren Führung (K) und der inneren Nadel (J) eine zusammenpassende gekrümmte Geometrie aufweisen.

**Revendications**

1. Dispositif utilisé pour établir rapidement un shunt portosystémique intrahépatique transjugulaire en localisant une veine porte dans un foie pour recevoir le shunt, comprenant :

un guide externe courbé creux allongé (K) ;
une poignée externe (A) ayant une lumière interne (E) et un premier adaptateur luer lock (D), la poignée externe (A) étant attachée à une extrémité proximale du guide externe (K), le guide externe (K) étant en communication d'écoulement avec la lumière interne (E) et le premier adaptateur luer lock (D) ;
une aiguille interne courbée creuse allongée (J) ;
un embout (F) ayant un deuxième adaptateur luer lock (I), l'embout (F) étant attaché à une extrémité proximale de l'aiguille interne (J), l'aiguille interne (J) étant en communication d'écoulement avec le deuxième adaptateur luer lock (1) ;
dans lequel l'aiguille interne (J) est reçue de manière coulissante à travers le premier adaptateur luer lock (D) et dans le guide externe (K) à travers la lumière interne (E) ;
dans lequel l'aiguille interne (J) est configurée pour tourner à l'intérieur du guide externe (K) par la manipulation de l'embout (F) ;
dans lequel une pointe distale de l'aiguille interne (J) est déployée hors et au-delà d'une pointe distale du guide externe (K), la pointe distale de l'aiguille interne (J) étant configurée pour être avancée dans le parenchyme du foie afin de localiser la veine porte ; et
dans lequel la poignée externe (A) a des prises pour doigts (B) le long de son bord inférieur afin de permettre à un opérateur de donner une torsion au guide externe, le dispositif comprenant en outre des ailettes pour doigts (C) sur la poignée externe (A) pour aider au déploiement de l'aiguille interne (J).

2. Dispositif selon la revendication 1, dans lequel l'aiguille interne est capable d'aspirer un fluide à travers celle-ci.

**3.** Dispositif selon la revendication 1, comprenant en outre une coiffe d'embout (G) reçue de manière détachable sur l'embout (F).

**4.** Dispositif selon la revendication 1, comprenant en outre un marqueur (H) sur l'embout (F) fournissant une indication de l'orientation de l'aiguille interne (J).

**5.** Dispositif selon la revendication 1, dans lequel les pointes distales du guide externe (K) et de l'aiguille interne (J) ont une géométrie courbée assortie.

*FIG. 1*

*FIG. 2*

FIG. 3

FIG. 4

201

203

202

FIG. 5

FIG. 6

211

209

212

209

FIG. 7

# FIG. 8

FIG. 9

FIG. 10

*FIG. 11*

*FIG. 12*

224

228

225

227

221

222

226

EP 2 994 177 B1

FIG. 13

EP 2 994 177 B1

FIG. 14

FIG. 15

K

FIG. 16

EP 2 994 177 B1

FIG. 17

FIG. 18

FIG. 19

## REFERENCES CITED IN THE DESCRIPTION

**Patent documents cited in the description**

- US 2012283640 A1